(19) Europäisches Patentamt
European Patent Office
Office européen des brevets

(11) Publication number: 0 035 065
B1

(12) EUROPEAN PATENT SPECIFICATION

(45) Date of publication of patent specification: 25.09.85

(21) Application number: 80108026.8

(22) Date of filing: 18.12.80

(51) Int. Cl.[4]: C 08 J 3/24, B 29 C 71/02, B 29 C 71/04, A 61 L 33/00 // C08L27/06

# (54) A crosslinked polyvinyl chloride sheet, a method for producing the same and the use thereof.

(30) Priority: 26.02.80 JP 22270/80

(43) Date of publication of application: 09.09.81 Bulletin 81/36

(45) Publication of the grant of the patent: 25.09.85 Bulletin 85/39

(84) Designated Contracting States: BE DE FR GB IT

(56) References cited:
GB-A-2 009 359
GB-A-2 025 895
GB-A-2 025 981
GB-A-2 027 038

(73) Proprietor: TORAY INDUSTRIES, INC.
2, Nihonbashi Muromachi 2-chome Chuo-ku
Tokyo 103 (JP)

(72) Inventor: Hatada, Kenji
A1-23, 10 Sonoyama 2-chome
Otsu-shi Shiga-ken, 520 (JP)
Inventor: Kobayashi, Hiroaki
B3-24, 15 Sonoyama
2-chome, Otsu-shi Shiga-ken, 520 (JP)

(74) Representative: Kador . Klunker . Schmitt-Nilson . Hirsch
Corneliusstrasse 15
D-8000 München 5 (DE)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to polyvinyl chloride sheets and the use thereof for blood and infusion bags. The surface of the sheets is modified such that the diffusion of plasticizer to the surface is suppressed and compatibility to the contents of the bags is improved.

It is well known that a thin crosslinked layer on the surface of chlorine containing vinyl polymers, such as polyvinyl chloride and polyvinylidene chloride formed by glow discharge or UV radiation, acts as a barrier for lower molecular weight substances such as monomer, plasticizer and additives to diffuse to the surface. This surface modification is considered as advantageous, because one of the most serious defects of such resins is their hardening and the environmental contamination caused by the diffusion and release of the plasticizer.

It has been surprisingly found that the surface becomes rough and loses transparency when the polyvinyl chloride sheet with a thin crosslinked layer is heated above its softening point. The transparency of the sheet is, however, important for many applications. For example, if medical bags, such as blood bags and infusion bags, lose their transparency during the heating process of autoclave sterilization or ethylene oxide gas sterilization, the contents cannot be observed any longer from the outside.

At present most blood bags are made of polyvinyl chloride sheet because of its flexibility and other well-balanced properties. However, the problem of compatibility, the decrease in number and the activity of the platelets during preservation still remains unsolved.

Usually the surface becomes hydrophillic (wettable) when treated by glow discharge, which may be good or bad depending on the application in question. In the use for blood bags and infusion bags, a lower diffusion rate of water is preferable. The glow discharge treatment heretofore carried out has not produced noteworthy effects in this regard.

Furthermore, it has been found that an emulsified lipid contained in a high calorie infusion increases in its size, which may be caused by the interaction between emulsion and hydrophillic surface treated by normal glow discharge.

It is therefore the object of the present invention to provide an improved polyvinyl chloride sheet material which is particularly suitable for blood and infusion bags, which is particularly transparent, even when subjected to a sterilization process, and which has improved preservation qualities for platelets and bio-compatibility and a retarded diffusion of plasticizer.

The polyvinyl chloride sheet of the present invention is characterised in that at least one surface of the sheet is crosslinked to an extent of thickness of 0.05 to 2 µm, the degree of chlorination of the cross-linked surface is not greater than 45% compared to the non-crosslinked sheet, and the heat shrinkage of the sheet is not greater than 1%. The sheet or film of the present invention consists of polyvinyl chloride as a main constituent and may contain additives such as plasticizer and anti-oxidants, depending on the application in question.

The crosslinked surface or surface layer is 0.05 µm to 2 µm thick and the degree of chlorination compared to the non-crosslinked sheet is not greater than 45%, more preferably 0.1 to 1 µm thick, and not greater than 30% of the original chlorination degree.

The degree of chlorination can be measured quantitively by such methods as molecular analysis of the chlorine atoms, XPS (X-ray photo electron spectroscopy) analysis and XMA (X-ray micro analysis). Molecular analysis is carried out by completely burning the samples in a oxygen flask forming chlorine gas, which is absorbed in a hydrazine solution and titrated sign a potentiometer and silver nitrate. In XPS analysis, the degree of chlorination is measured by the peak area ratio of $C_{1s}$ and $Cl_{2p} \cdot (Cl_{2p}/C_{1s})$. In XMA analysis, the chlorine concentration can be measured directly. These three methods have been tried for crosslinked samples and non-crosslinked (original) polyvinyl chloride samples, and it has been found that similar values of the degree of chlorination (the ratio of crosslinked layer to the original) can be measured by these three methods. The crosslinked part and the non-crosslinked part can be easily separated by solving in tetrahydrofuran.

Concerning the thickness of the crosslinked layer, a thickness of less than 0.05 µm is believed to act insufficiently as a barrier for low molecular weight substances. On the other hand, if the thickness is greater than 2 µm, the crosslinked layer gels harden and a small degree of mechanical deformation of a treated sheet causes the formation of micro cracks, through which small molecular weight substances can freely diffuse. When the polyvinyl chloride sheet with a crosslinked layer of more than 2 µm in thickness is solved in tetrahydrofuran, yellowish needle-like undissolved substances can be observed as they separate from the bulk layer right after the submersion of the sample.

The thickness of the crosslinked layer can be measured with an electron microscope. A sample is prepared by dyeing the sheet with $OsO_4$ and slicing it after a conventional packing procedure. The crosslinked layer consists of two layers, i.e. the undyed layer on the top surface and the dyed layer. We claim in this invention that the thickness is the sum of the above two layers. The thickness can be more easily estimated by weighing the gel mass of a specific area, assuming its specific gravity is 1.2. This method is, however, not satisfactorily correct, because the specific gravity largely depends on the amount of additives and the degree of crosslinking. On the basis of experimental results, this "gel weight" method has an error of about 20% compared to the electron microscope method, but despite this, it is a very convenient and easy method.

The polyvinyl chloride sheet is not necessarily covered wholly by the crosslinked layer. The sheet can be treated partly or locally, depending on the desired purpose of its use.

The heat shrinkage of the sheet may not be greater than 1%. If the heat shrinkage is more than 1%, the cross-linked surface layer shows a striped pattern caused by uneven deformation after specific thermal history. This phenomenon accompanied by loss of transparency probably originates from the different thermal shrinkage (irreversible thermal dimensional change) of the crosslinked and non-crosslinked layers. For a sheet with a heat shrinkage of not more than 1%, the formation of a striped pattern is much reduced, so that transparency remains as it was before the treatment. For a sheet with less than 0.5%, apparent change cannot be observed even with a microscope. Thus, in order to maintain the transparency of the treated sheet, the sheet should have a heat shrinkage of not more than 1%, and more preferably of not more than 0.5%.

The heat shrinkage is measured as follows. Three sheet samples are taken from a treated polyvinyl chloride sheet as shown in Figure 1, and marks A to H are provided on the sample sheets so that the distances between AF, BG, CH and AC, DE, FH are 100 mm. These samples are heated in an air circulating oven at 100±2°C for 10 minutes and are then taken out and cooled to room temperature. The lengths AF, BG, CH and AC, DE, FH are measured. The longitudinal distance between the marks, 1 (mm), is calculated as an average of AF, BG, and CH, and the longitudinal heat shrinkage S(%) is calculated as follows:

$$S=\frac{100-1}{100}\times 100.$$

Similarly, by taking an average of AC, DE, FH, the transverse distance 1 (mm) leads to a transverse heat shrinkage S (%).

The barrier effect of the glow discharge treatment is measured as follows. An extraction apparatus is placed on the polyvinyl chloride sheet with a polytetrafluoroethylene sheet ring disposed therebetween. The apparatus is filled with 13.6 g (20 $cm^3$) n-hexane having an 11.34 $cm^2$ extraction area and is kept at 40°C for two hours. DOP (dioctyl phthalate or di-2-ethyl hexyl phthalate), which is the most commonly used plasticizer for polyvinyl chloride, which is extracted in n-hexane, is measured quantitively by gas chromatography.

It has also been found that the crosslinked layer is partly fluorinated when a gas comprising fluorine atoms is mixed in a low discharge gas. Fluorinated crosslinked surfaces show hydrophobic properties reducing the penetration coefficient of moisture as well as reducing the diffusion coefficient of plasticizer. To achieve this effect it is preferable that the number of fluorine atoms to carbon atoms be not less than 10% on the surface of the sheet.

Of course the polyvinyl chloride sheet is not necessarily completely covered by the crosslinked layer. By way of example, the blood bag may be designed so that the inside surface is modified for improving biocompatibility and the outside for preventing moisture penetration by fluorination.

The process of the invention comprises heat treatment of the polyvinyl chloride sheet to reduce its heat shrinkage to not more than 1%, and exposure of the sheet to a glow discharge at a pressure of $10^{-2}$ to 10 torr and at an electric supply frequency of 1 to 1000 kHz. The heat treatment is usually carried out by means of hot rolls.

One example of the glow discharge treatment is illustrated in Figure 2. In this Figure, 1, 2, 3 are the web transportation roll (earth), the web feeding roll and the winding roll respectively. 6, 9, 10 are a glass-coated discharge electrode (rod-like), a matching network and a high-voltage power generator respectively. 8 is the low discharge equipment case containing 1, 2, 3, 6. 4 is a glow discharge gas inlet and 5 is an outlet. 7 is the sheet to be treated.

First the sheet is fed to the feeding roll 2, then the equipment is evacuated by a vacuum pump through 5, and the specific gas is introduced into the apparatus while maintaining the glow discharge pressure at a predetermined level. Then the glow discharge is initiated by applying a high voltage between the electrode 6 and the roll 1. The sheet is fed from 2 and wound up by 3 via roll 1, where the sheet surface is exposed to the glow discharge forming a crosslinked thin layer on the surface.

The glow discharge pressure is between $10^{-2}$ and 10 torr. As glow discharge gas, a gas mixture is preferable which consists of carbon monoxide and at least one gas selected from Ar, $N_2$, $CO_2$, $CF_4$, water etc. A gas mixture of CO and $CF_4$ is particularly preferred, because the moisture penetration coefficient as well as the diffusion coefficient of plasticizer are reduced.

The gas mixture of CO and $CF_4$ may contain 10 to 90 mol% CO, and preferably 40 to 60 mol% CO.

The electric frequency for this treatment is 1 to 1000 kHz, preferably 1 to 400 kHz.

There are two types of glow discharge treatment, one using an outside electrode and the other using an inside electrode. In the former type the glow discharge is maintained by a magnetic or electric field induced by electrodes such as a coil or a couple of plates attached to the outside of the equipment, usually using a high frequency of 13.56 MHz. In the latter type, the electrodes are placed inside the equipment, and relatively lower frequencies can be applied to maintain the discharge.

It has very surprisingly been found that the polyvinyl chloride sheet is much more effectively treated by the inside electrode method than the outside electrode method. The surface treated by the inside electrode

is obviously highly crosslinked forming a good barrier in a much shorter period of treatment, which can easily be observed by both the tetrahydrofuran solution test and gas permeation measurement. These results clearly show that the inside electrode method is much more preferable for the process of this invention.

The fluorination of the crosslinked layer can be accomplished during or after the crosslinking treatment. The addition of fluorine gas or gaseous fluorinated (halogen) hydrocarbons to the glow discharge gas is probably the easiest way to fluorinate the cross-linked surface. Generally after the glow discharge treatment, the formation of the groups such as —OH, =CO, —COOH, making the surface hydrophillic, can be observed by ESCA and infrared absorption spectroscopy (ATR method). The polyvinyl chloride sheet surface becomes wettable by the glow discharge treatment which is also true for other polymers. However, a glow discharge treatment using fluorine gas, fluorinated hydrocarbons or other fluorine-containing gas mixtures makes the surface hydrophobic and crosslinked. Moreover, while the polyvinyl chloride sheet yellows by usual glow discharge treatment, the sheet does not yellow by the addition of fluorine-containing gas to the glow discharge gas.

The glow discharge in fluorine and other halogen-substituted hydrocarbon gases, such as $CFClH_2$, is unstable and often emits uncontrollable heat. Especially when chlorine atoms are present, decomposed products giving off a bad odour are formed during the glow discharge. On the other hand, the glow discharge in fluorine-substituted hydrocarbons, such as $CF_4$, $CHF_4$, and $CH_3CHF_2$ is very stable and shows excellent treatment efficiency. The gases containing an unsaturated bond, such as $C_2F_4$ and $C_2H_2F_2$, are, however, not preferable because they polymerize in the glow discharge environment. Fluorine gas has some disadvantages such as difficulty in handling because of its high reactivity and poorer treatment efficiency compared to fluorinated hydrocarbons.

Diluting fluorine gas and fluorinated hydrocarbons with other inert gases, such as CO, Ar, $N_2$, $H_2$, $O_2$ He and Ne, shows no negative effects. For example, the treatment with a $CO/CF_4$ mixture gas shows almost the same effects as with $CF_4$ gas alone in respect of surface tension change and the reduction of plasticizer diffusion, but shows a superior effect in respect of the preservation of blood platelets.

Consequently, for fluorinating the surface, fluorinated hydrocarbons containing no chlorine, such as $CF_4$ and the mixtures of said gas with inert gases, are most preferable. Other inert gases may be selected so as to produce the required surface properties. The surface of the polyvinyl chloride treated by the above-mentioned glow discharge can be analysed by ESCA. In the case of fluorinated hydrocarbons containing gas, the peaks, such as

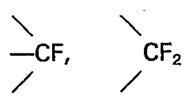

and $—CF_3$ are observed, while in case of fluorine gas no $—CF_3$ peak, the most effective group as regards its hydrophobic property, is observed, which may be the reason why the treatment efficiency of the fluorinated hydrocarbons is more satisfactory.

The fluorinated crosslinked thin layer on the surface of the polyvinyl chloride acts as a good barrier in respect not only of DOP diffusion to the surface but also the diffusion of the contents in the bags to the outside because of low chemical affinity between the surface and the contents. Therefore this kind of treatment is especially suitable for medical purposes, such as for blood bags and infusion bags.

For blood bags, polyvinyl chloride sheet is used exclusively because of its superior mechanical properties. However, as is well known, many difficulties, such as adhesion and agglomeration of platelets and gelation and coagulation of plasma, take place when blood is contacted with a foreign surface. When using polyvinyl chloride blood bags of the present art, platelets coagulate on the surface, reducing the number of active platelets to 60 to 70% of the initial number after 10 hours of storage. The development of blood bags which can preserve blood for longer periods as fresh as possible is therefore a long-felt want in this field.

Usually anti-coagulation agents are added to blood bags to prevent blood coagulation. These agents are classified into four types. One of these comprises so-called ACD liquids containing sodium citrate as a main constituent, citric acid and dextrose. Another comprises the CPD liquid which contains sodium phosphate in addition, as compared to the ACD liquid. The others are heparin and the EDTA liquid which is a potassium salt of ethylene-diamine-tetraacetate.

During the observation of platelets adhesion and coagulation behaviour to a glow discharge treated polyvinyl chloride sheet by a scanning electron microscope, it was found that the sheet shows an extremely favourable effect on platelet preservation, only when ACD or CPD liquids, which contain sodium citrate as a main constituent, are used as blood preservation agents. When heparin is used as a blood preservation agent, adhesion and coagulation of many platelets and exsertion of pseudo-podium are observed on the treated polyvinyl chloride sheet, which is comparable to the case of an untreated polyvinyl chloride sheet.

Using ACD liquid, the platelets' adhesion and coagulation to the treated sheet is negligible and exertion of pseudo-podium is not observed. Of course if the polyvinyl chloride is not glow discharge treated, even though ACD liquid is used, many platelets adhere and coagulate on the sheet surface so that said surface is almost covered.

The reason for the combination of a glow discharge treated polyvinyl chloride sheet and blood preservation agents containing sodium citrate showing this very unique and noteworthy effect on blood compatibility has not yet been elucidated, but it is possible that the surface may be modified by glow discharge treatment so as to be compatible with sodium citrate.

The properties of a treated polyvinyl chloride sheet surface naturally depend on the glow discharge gases. Consequently, the preservation of platelets also depends on the glow discharge gases. The use of different gases leads to the result that a polyvinyl chloride sheet treated using glow discharge gases, which comprise carbon monoxide and at least another gas selected from Ar, $N_2$, $CO_2$ and $H_2O$, shows an excellent effect for platelet preservation. The gas mixture of CO and $H_2O$ is especially preferable for this purpose.

On the other hand, glow discharge treatment using CO gas alone shows a better effect compared with the untreated sheet, and is much inferior to the above-mentioned treatment with gas mixtures.

Though the mixture ratio of CO and other gases selected from Ar, $N_2$, $CO_2$, $H_2O$ is dependent on parameters such as discharge stability and surface properties to be modified, the amount of CO is preferably more than 10 mol% for forming a good crosslinked thin layer as a diffusion barrier for the plasticizer.

The blood bags must be glow discharge treated at least on the inside surface of the bag, but both sides can be treated. Usually a label indicating the anti-coagulation agent and blood constituents and other information is attached to the outside surface of the bag, and sometimes some constituents of the adhesive of the label are suspected to diffuse through the sheet and contaminate the blood. When glow discharge treated on both surfaces, the blood bag has the advantage that it eliminates this kind of suspicion. For the glow discharge treatment of the outside surface of the bag, the gases are not limited as specified for the inside surface treatment, and CO, Ar or gas mixtures with other gases can be used.

It is usually required that carbon dioxide formed in the blood can diffuse out of the blood bag. The glow discharge treated blood bags have almost the same $CO_2$ permeation coefficient as the untreated bags.

The manufacturing process of the blood bags can be performed in several ways. By way of example, the glow discharge treated sheet is welded into bags, followed by sterilization and the introduction of the anti-coagulation agent into the bags. Sterilization may be carried out by ethylene oxide, radiation or autoclave treatment, because the crosslinked layer formed by glow discharge raises the heat resistence of the sheet.

As described above in detail, the blood bag is glow discharge treated at least on its inside surface in the presence of a mixture of gases comprising CO and at least one other gas selected from Ar, $N_2$, $CO_2$ and $H_2O$. Other gases which act as diluents can be added to this discharge gas. This blood bag with anti-coagulation agent has the great advantage over the untreated bag and the bag treated with gases other than those specified above, namely that the lifetime of the platelets is extended and the number of floating platelets is slowly reduced. Other advantages are that the original mechanical properties are not changed at all after treatment because of the ultra thin crosslinked layer and that the solution of the plasticizers into the blood is drastically reduced.

The blood bag is most suitable for a bag for platelets preservation, a so-called triple bag. However, because the platelets are contained in total blood and other blood constituents, this invention is very useful even for so-called single bags and double bags. If the inside of the tubes attached to such bags is glow discharge treated with the above specified gases, the total system of the blood bags becomes more preferable in respect of platelet preservation.

The polyvinyl chloride sheet in accordance with this invention provides the following superior effects and properties:

(1) The sheet does not loose its transparency after longterm thermal aging, which is a very important advantage in the use of blood bags and infusion bags.
The sheets of the prior art, which are glow discharge treated, gradually loose their transparency because of the difference of irreversible dimension stability between the crosslinked layer and the non-crosslinked layer.

(2) Emulsions contacting the sheet surface remain very stable in respect of the particle size. If the emulsifying lipid in high calorie infusions increases in particle size, this may lead to obliteration of the blood vessels. Therefore said sheets are especially suitable for high-calorie infusion bags.

(3) The sheets are suitable for medical bags because the plasticizer diffusing into the contents and water diffusing out of the bags are drastically suppressed.

(4) The sheet has the very special property of elongating the active life of blood platelets contacting it, thereby providing high-quality blood bags.

As stated above the sheets of this invention are especially useful for bags in the medical field. Because of their unique properties, the sheets have, however, a very wide range of other applications, such as in the packing of food and medicines, in interior and exterior decorating and in agricultural use, such as sheets for greenhouses where transparency and plasticizer diffusion are very important points for the application of polyvinyl chloride sheets.

The invention will now be illustrated by way of non-limiting examples.

Example 1

The glow discharge apparatus shown in Figure 2 was employed to treat plasticized, commercially available 320 µm polyvinyl chloride sheets which contained 41 phr of DOP by weight. After the sheets were placed into the apparatus, the latter was degassed. Then carbon monoxide gas was introduced into the apparatus and the pressure was maintained at 1.33 mbar (1 torr). The sheets were subjected to a glow discharge generated from the 110-kilocycle per second high voltage oscillator at 900 W of discharge power, at various sheet speeds.

DOP migrating from the sheet to n-hexane was measured by the method described hereinbefore. Sections cut from the sheets were extracted with an excess of tetrahydrofuran at its boiling point for a period of 2 hours. The solvent insoluble residue was recovered, washed with hot solvent and carefully dried. Then the contents of chlorine of the solvent insoluble residue were measured by elementary analysis.

Thereafter the sections cut from the sheet were dyed with osmic acid and the thickness of the crosslinked layer was measured with an electron microscope.

These results are shown in Table I and Figure 3. In Figure 3, curve A expresses the DOP content of n-hexane and curve B expresses the ratio of the chlorine contents of the crosslinked layer.

The appearance of the crosslinked layer stated in Table I are that of the solvent insoluble residue. These were observed with the naked eye.

TABLE I

| Sheet speed (m/min) | Thickness of crosslinked layer (µm) | Ratio of chlorine content (%) *1) | DOP content of n-hexane (%) | Appearance of crosslinked layer |
|---|---|---|---|---|
| 0.1 | 2.9 | 6.9 | 60 | yellow needle crystal |
| 0.2 | 1.4 | 9.0 | 0.4 | yellow thin film |
| 0.5 | 0.91 | 9.7 | 0.1 | pale-yellow thin film |
| 0.6 | 0.72 | 11.1 | 0.1 | pale-yellow thin film |
| 0.8 | 0.58 | 12.5 | 0.1 | pale-yellow thin film |
| 1.0 | 0.51 | 13.9 | 0.1 | pale-yellow thin film |
| 1.5 | 0.45 | 16.7 | 0.1 | pale-yellow thin film |
| 2.0 | 0.35 | 18.1 | 0.1 | pale-yellow thin film |
| 2.5 | 0.29 | 19.4 | 0.1 | pale-yellow thin film |
| 3.0 | 0.27 | 20.8 | 0.1 | pale-yellow thin film |
| 4.0 | 0.18 | 22.9 | 0.4 | pale-yellow thin film |
| 5.0 | 0.09 | 25.0 | 5.0 | ple-yellow thin film |
| 6.0 | 0.04 | 53.6 | 30.0 | white thin film, slightly swollen |
| 7.5 | —— | —— | 57.0 | white very thin film |
| Untreated | 0 | 100 | 100 | no crosslinked layer |

*1) The ratio of chlorine content of the crosslinked layer to that of the non-crosslinked layer.

In order to reduce the migration of DOP to under 1/10 compared to that of untreated sheets, Table I and Figure 3 indicate that the ratio of the chlorine contents must be under 45% and the thickness of the crosslinked layer must be from 0.05 µm to 2 µm. To reduce the migration of DOP to under 1/100, the ratio of chlorine contents must be under 30% and the thickness of the crosslinked layer must be from 0.1 µm to 1 µm.

Thereafter untreated sheets of the same polymer as above were heatset at 150°C for various times.

Then the sheets were subjected to glow discharge under the same condition as described above, except that the sheet speed was 3 m/min.

The sheets were heatset again under the given conditions to measure the heat shrinkage ratio as described hereinbefore. The heat shrinkage ratio of the sheets and the amount of DOP extracted with n-hexane from sections cut from the sheet were measured and the transparency of the sheets and the existance of waving on the surface of the sheets were observed. These results are shown in Table II.

The results indicate that the sheets with a heat shrinkage ratio of under 1% will maintain their transparency, even when heated.

Example 2

After heatsetting a plasticized 510 μm polyvinyl chloride sheet at 150°C for 2 minutes, the sheet was subjected to glow discharge under the same conditions as in Example 1, except that the sheet speed was 2 m/min. The sheet was then heatset again under the given conditions to measure the heat shrinkage ratio as described hereinbefore. The amount of DOP extracted with n-hexane from the sheet was also measured by gas chromatography. The transparency of the sheets was observed by the naked eye and the presence of waving on the surface of the sheet was checked using a microscope. These results are shown in Table III.

Example 3

Commercially available plasticized 320 μm polyvinyl chloride sheets containing 41 phr of DOP by weight, were treated as described in Example 1, except for the conditions of discharge power and sheet speed, using various high voltage power supplies. Each of these power supplies delivered an alternating current of 60, 500, 1 kilo, 5 kilo, 9 kilo, 20 kilo, 110 kilo, 400 kilo, and 13.56 mega cycles/s. The discharge power was 700 W and the sheet speed was 2 m/min.

To generate the glow discharge, two types of electrodes were used. One was a copper pipe with an outer diameter of 10 mm, cooled with water circulating in the pipe. The other was a copper pipe covered with glass and also cooled with water circulating in the pipe with the same outer diameter of 10 mm.

The amount of DOP extracted with n-hexane from sections cut from the treated sheets was measured by gas chromatography. The results are illustrated in Figure 4. Curve A indicates the results using the copper pipe as electrode and Curve B indicates the results using the copper pipe covered with glass.

Thereafter, sections cut from the sheets were extracted with an excess of tetrahydrofuran at its boiling point for a period of 2 hours. The solvent insoluble residue of the sheets subjected to glow discharge generated by the high voltage power supply of a frequency of not greater than 500 cycles per second and 13.5 mega-cycles per second were milk-white thin films which were slightly swollen with tetrahydrofuran. The solvent insolvent residue of the sheets subjected to glow discharge generated by the high voltage power supply of from 1 kilo-cycle to 400 kilo-cycles were pale yellow thin films and were not swollen with tetrahydrofuran.

The phenomena described above and the results shown in Figure 4 imply that the crosslinked layer of the sheets treated with high voltage power supply of frequencies of not greater than 500 cycles per second and 13.56 mega-cycles per second are not so highly cross-linked and thus DOP can migrate through the crosslinked layer from the inside of the sheet to n-hexane.

The results shown in Figure 4 indicate that the optimum frequency of the power supply is from 1 kilo-cycle to 1 mega-cycle per second, and more preferably the optimum frequency is from 1 kilo-cycle to 400 kilo-cycles per second. The results shown in Figure 4 also indicate that the electrode covered with glass is a more preferable electrode to generate glow discharge.

TABLE II

| Heatset time (s) | Subjected to gas discharge | Heat shrinkage ratio (%) | Transparency | Presence of waving | Amount of DOP permeating through unit area of sheet into n-hexane (μg/cm²) |
|---|---|---|---|---|---|
| 0 | no | 7 | transparent | absent | 1287 |
| 0 | yes | 6 | opaque | present | 7.1 |
| 10 | yes | 4 | opaque | present | 8.0 |
| 30 | yes | 2 | opaque | present | 8.0 |
| 40 | yes | 1 | slightly opaque | can be slightly observed | 7.0 |
| 60 | yes | 0.5 | transparent | absent | 7.0 |
| 90 | yes | 0 | transparent | absent | 7.8 |

TABLE III

| | Heatset | Subjected to glow discharge | Heat shrinkage ratio (%) | Transparency | Presence of waving | Amount of DOP permeating through unit area of sheet (μg/cm²) |
|---|---|---|---|---|---|---|
| Control sheet | no | no | 5 | transparent | absent | 1675 |
| Control sheet | no | yes | 4 | opaque | present | 0.2 |
| Sheet according to the invention | yes | yes | 0 | transparent | absent | 0.2 |

Example 4

Medical grade plasticized 400 μm polyvinyl chloride sheet, containing 35% of DOP by weight was treated as described in Example 1 under the conditions of a pressure of 0.799 mbar (0.6 torr), a discharge power of 900 W and a sheet speed of 1 m/min, using a mixed gas consisting of $CF_4$ (40 mol%) and CO (60 mol%).

A sheet of the same polymer was also treated as described above, but pure CO gas was used. Sections cut from these sheets were extracted with an excess of tetrahydrofuran at its boiling point for a period of 2 hours. The solvent insoluble residue was recovered, washed with hot solvent and dried. The solvent insoluble residue of the sheet subjected to the CO gas discharge was a yellow thin film, but that of the sheet subjected to the mixed gas discharge was a white thin film.

Small infusion bags were made from these sheets and from a sheet which was not subjected to glow discharge. These bags were filled with n-hexane and stored in a constant temperature box of 40°C for 2 hours. Then the amount of DOP in n-hexane in each bag was measured by gas chromatography. The results are shown in Table IV.

Other bags were filled with water of constant weight and then weighed. These bags were stored in a room at a constant temperature of 20°C and constant relative humidity of 65%, for two weeks. Then these bags were weighed again. The weight loss of water in the bags was calculated from these data. The results are also shown in Table IV.

TABLE IV

| Sheet material | Amount of DOP permeating through unit area of sheet (μg/cm$^2$) | Weight loss of water in the bag (weight %) |
|---|---|---|
| Control sheet | 3921 (100%) | 5.7 |
| Sheet subjected to CO gas discharge | 175 (4.4%) | 4.4 |
| Sheet subjected to CO—$CF_4$ mixed gas discharge | 122 (3.1%) | 0.19 |

The results shown in Table IV indicate that the amounts of DOP and water diffusing through the sheet subjected to CO—$CF_4$ mixed gas discharge are smaller than those of the control sheet.

Example 5

A sheet of the same polymer as used in Example 4 was treated as described in Example 4, but using a mixed gas consisting of $CF_4$ (60 mol%) and CO (40 mol%). Furthermore, a sheet of the same polymer was treated as described in Example 4, but using CO gas.

Small infusion bags were made from these sheets and a control sheet, and these were sterilized with ethylene oxide gas.

These bags were filled with an emulsified lipid commercially available under the trade mark Intralipid 10% from Green Cross Co., Japan, and then stored in a room for 72 hours. Then the DOP content of the emulsified lipid was measured by the procedure described below. 40 cm$^3$ of n-hexane were added to 10 cm$^3$ of the emulsified lipid, the mixture was shaken, then the n-hexane was separated from the emulsified lipid and the same procedure was repeated twice. Then all of the n-hexane was dehydrated and distilled off at reduced pressure. 10 cm$^3$ of n-hexane containing dioctyl phthalate, as internal standard materials, were added to the residue and the DOP content of the mixture was measured by gas chromatography. The results are shown in Table V.

The particle diameter of the emulsified lipid in the bags was measured by the procedure described below. 150 cm$^3$ of water were added to 0.1 cm$^3$ of the emulsified lipid in the bag and then 150 cm$^3$ containing 20% sodium chloride by weight were added. The number of particles of the emulsified lipid with a particle diameter of more than 1.5 μm was measured with a Coulter counter.

The particles of the emulsified lipid increased in size in the course of time after the water containing the sodium chloride was added, so that the measurements were completed within three minutes after the addition of the water containing the sodium chloride. The results are shown in Table V.

TABLE V

| Bag | DOP content in n-hexane (ppm) | Number of particles of emulsified lipid (number/0.5 cm$^3$) | |
|---|---|---|---|
| | | beyond 1.5μ | beyond 4μ |
| Bag made of control sheet | 6 | 24.392 | 13.0 |
| Bag made of the sheet subjected to CO gas discharge | not detectable | 14.857 | 9.7 |
| Bag made of the sheet subjected to CO—$CF_4$ mixed gas discharge | not detectable | 12.476 | 4.7 |

The results of Table V show that the amount of DOP diffused to n-hexane from the bag made of the sheet subjected to the CO—$CF_4$ mixed gas discharge is small, and the particles of the emulsified lipid hardly increase in size in that bag.

Example 6

Sheets cut from a blood bag made of plasticized polyvinyl chloride, commercially available under the trade mark Telflex BB-TO6OCJ from Telmo Co., Japan, were put on the drum in the glow discharge apparatus shown in Figure 2 and then subjected to Ar gas discharge under the conditions of a pressure of 0.6 torr, a discharge power of 1200 W and a sheet speed of 1 m/min.

Sections cut from the sheets were extracted with an excess of tetrahydrofuran at its boiling point for 2 hours. An insoluble thin film remained. The adhesion capacities of the treated sheet and a control sheet to water were measured. The adhesion capacity of the treated sheet was 118 dyne/cm and that of the control sheet was 91 dyne/cm.

9 $cm^2$ sections cut from the sheet subjected to the gas discharge and a control sheet were immersed in blood which consisted of 7 ml of blood from a carotid artery of a rabbit and 1.5 ml of an ACD solution in which 100 ml contained 2.20 g of sodium citrate, 0.80 g of citric acid, 2.20 g of dextrose and the remainder distilled water, and shaken at 100 rpm in a constant temperature box of 37°C for 30 min. Then the blood was rotated at 1200 rpm for 30 minutes and separated centrifugally. The number of platelets in the superanatant liquid was measured with a Coulter counter. The results are shown in Table VI. The number of platelets is expressed by the proportion to the number of platelets of the blood in which the control sheet was immersed, which is expressed as 100%.

TABLE VI

| Sheet | Ratio of the number of platelets (%) |
|---|---|
| Control sheet | 100 |
| Sheet subjected to gas discharge | 137 |

Then the rest of the blood containing the supernatant liquid was rotated at 3600 rpm and separated centrifugally. Then 5 ml of a physiological salt solution were added to 1 ml of the supernatant liquid of the blood. The absorbancy of the solution at 545 milli-micron was measured. The absorbancy of the blood in which the treated sheet was immersed was the same as that of the blood in which no sheet was immersed. Thus no hemolysis of the blood, in which the sheet subjected to gas discharge was immersed, was observed.

The sheets immersed in the blood were washed with 10 ml of a physiological salt solution five times, fixed with a solution consisting of 9% of formaldehyde and 91% of a physiological salt solution and then observed with a scanning electron microscope.

The control sheet was covered with a large number of platelets, while the sheet subjected to the gas discharge was covered with a small number of platelets only.

The results described above imply that a bag which is made of the plasticized polyvinyl sheet subjected to a gas discharge, and which contains an anti-coagulation agent comprising sodium citrate, is more suitable for blood bags.

Example 7

9 $cm^2$ sections cut from a sheet treated as described in Example 6 and a control sheet were immersed in 7 ml of blood which consisted of blood from a carotid artery of a rabbit and 6 units per ml of heparin and shaken at 100 rpm in a constant temperature box of 37°C for 30 minutes.

The number of platelets in the blood was measured by the method described in Example 6. The results are shown in Table VII.

TABLE VII

| Sheet | Ratio of the number of platelets (%) |
|---|---|
| Control sheet | 100 |
| Sheet subjected to gas discharge | 106 |

Hemolysis was investigated by the method described in Example 6. No hemolysis of the blood was observed in which the sheet subjected to gas discharge was immersed.

The sheets fixed in the same way as described in Example 6 were observed with a scanning electron microscope. Both the control sheet and the sheet subjected to gas discharge were covered with a large number of platelets and a part of the platelets adhering to the sheets aggregated and exertion of pseudo-podium was observed on both sheets.

The results described above imply that heparin as an anti-coagulant agent is less suitable for blood bags made of the polyvinyl chloride sheet subjected to a gas discharge.

Example 8

Medical grade plasticized polyvinyl chloride sheets, containing 35% of DOP by weight, were treated as described in Example 1, but at a sheet speed of 1.2 m/min.

Blood which consisted of 91% of blood from a carotid artery of a rabbit and 9% of an anticoagulation agent by weight consisting of 3.8% of sodium citrate and 96.2% of physiological salt solution by weight, was shaken at 1200 rpm and separated centrifugally to make a platelet-enriched plasma which is the supernatant liquid.

4 $cm^2$ sections cut from a sheet subjected to gas discharge and a control sheet were immersed in the platelet-enriched plasma, stored in an incubator filled with carbonic acid gas at a constant temperature of 37°C for 2 hours, then washed with a solution which consisted of 10% of phosphate buffer having a pH value of 7.4 and 90% of physiological salt solution, fixed with the solution consisting of 9% formaldehyde and 91% physiological salt solution, and observed with a scanning electron microscope.

The control sheet was completely covered with a large number of platelets and most of the platelets aggregated. But platelets covered only a small part of the sheet subjected to gas discharge and only a few platelets were aggregated.

The results imply that sodium citrate as an anticoagulation agent is suitable for a bag made of the polyvinyl chloride sheet subjected to gas discharge. The results of this Example and Example 8 further imply that a bag which is made of a polyvinyl chloride sheet subject to gas discharge and containing an anticoagulation agent comprising sodium citrate, is suitable for whole blood and components of blood.

Example 9

A sheet of the same polymer as used in Example 8 was formed without heatsetting. The heat shrinkage ratio was 3%.

The same sheet with a heat shrinkage ratio of 0.3% as used in Example 8 and the sheet described above were treated as described in Example 8.

Small blood bags were made of these sheets, filled with 10 ml of ACD solution, sterilized in an autoclave at 121°C for 25 minutes an then allowed to cool.

The bag made from the sheet with a heat shrinkage ratio of 0.3% was transparent enough, so it was easy to check turbidity and the presence of suspended solids in the ACD solution in the bag. But the bag made from the sheet with a heat shrinkage ratio of 3% was too opaque, so it was very difficult to check turbidity and the presence of suspended solids in the ACD solution in the bag.

**Claims**

1. A crosslinked polyvinyl chloride sheet wherein

(a) at least one surface of the sheet is crosslinked to an extent of thickness of 0.05 to 2 μm,
(b) the degree of chlorination of the crosslinked surface is not greater than 45% compared to the non-crosslinked sheet, and
(c) the heat shrinkage of the sheet is not greater than 1%.

2. A crosslinked polyvinyl chloride sheet according to claim 1 wherein at least one surface of said sheet is crosslinked to an extent of thickness of 0.1 to 1 μm.

3. A crosslinked polyvinyl chloride sheet according to claims 1 and 2 wherein the degree of chlorination is not greater than 30%.

4. A crosslinked polyvinyl chloride sheet according to either of claims 1 to 3 wherein the heat shrinkage of said sheet is not greater than 0.5%.

5. A crosslinked polyvinyl chloride sheet according to either of claims 1 to 4 wherein the crosslinked surface is partially fluorinated.

6. A crosslinked polyvinyl chloride sheet according to claim 5 wherein the number of fluorine atoms on the crosslinked surface is not less than 10% of the carbon atoms.

7. A process for producing a crosslinked polyvinyl chloride sheet comprising heat-treating a polyvinyl chloride sheet such that the heat shrinkage of said sheet is not greater than 1%, and subjecting said sheet to a glow discharge at a pressure of $10^{-2}$ to 10 torr using a frequency of 1 to 1000 kilo-cycles.

8. A process according to claim 7 wherein said glow discharge is effected in a gas mixture comprising carbon monoxide and carbon tetrafluoride.

9. Infusion bags and blood bags produced by using polyvinyl chloride sheets according to either of claims 1 to 6.

10. The use of the blood bag according to claim 9 wherein at least the inner surface of the bag is treated with cold plasma and contains an anticoagulation agent comprising sodium citrate in the bag.

**Patentansprüche**

1. Eine vernetzte Polyvinylchlorid-Folie, in der

(a) wenigstens eine Oberfläche der Folie bis zu einer Dicke von 0,05 bis 2 µm vernetzt ist,
(b) der Grad der Chlorierung der vernetzten Oberfläche nicht größer ist als 45%, verglichen mit der nichtvernetzten Folie, und
(c) die Wärmeschrumpfung der Folie nicht größer als 1% ist.

2. Vernetzte Polyvinylchlorid-Folie nach Anspruch 1, bei der wenigstens eine Oberfläche der genannten Folie bis zu einer Dicke von 0,1 bis 1 µm vernetzt ist.

3. Vernetzte Polyvinylchlorid-Folie nach den Ansprüchen 1 und 2, bei der der Chlorierungsgrad nicht größer ist als 30%.

4. Vernetzte Polyvinylchlorid-Folie nach irgendeinem der Ansprüche 1 bis 3, bei der die Wärmeschrumpfung der genannten Folie nicht größer ist als, 0,5%.

5. Vernetzte Polyvinylchlorid-Folie nach irgendeinem der Ansprüche 1 bis 4, bei der die vernetzte Oberfläche teilweise fluoriert ist.

6. Vernetzte Polyvinylchlorid-Folie nach Anspruch 5, bei der die Anzahl der Fluoratome auf der vernetzten Oberfläche nicht geringer ist als 10% der Kohlenstoffatome.

7. Verfahren zur Herstellung einer vernetzten Polyvinylchlorid-Folie, das die Wärmebehandlung einer Polyvinylchlorid-Folie auf eine Weise, daß die Wärmeschrumpfung der genannten Folie nicht größer ist als 1%, und das Unterwerfen der genannten Folie einer Glimmentladung bei einem Druck von $10^{-2}$ bis 10 Torr unter Anwendung einer Frequenz von 1 bis 1000 kh umfaßt.

8. Verfahren nach Anspruch 7, bei dem die genannte Glimmentladung in einer Gasmischung erzeugt wird, die Kohlenmonoxid und Tetrafluorkohlenstoff umfaßt.

9. Infusionsbeutel und Blutbeutel, hergestellt durch Verwendung von Polyvinylchlorid-Folien nach irgendeinem der Ansprüche 1 bis 6.

10. Verwendung des Blutbeutels nach Anspruch 9, wobei wobei wenigstens die Innenoberfläche des Beutels mit einem kalten Plasma behandelt ist und in dem Beutel ein Antikoagulationsmittel enthalten ist, das Natriumcitrat umfaßt.

**Revendications**

1. Feuille de polychlorure de vinyle réticulée dans laquelle

(a) au moins une face de la feuille est réticulée sur une épaisseur de 0,05 à 2 µm,
(b) le degré de chloration de la face réticulée n'est pas supérieur à 45%, par comparaison avec la feuille non réticulée, et
(c) le retrait thermique de la feuille n'est pas supérieur à 1%.

2. Feuille de polychlorure de vinyle réticulée selon la revendication 1, dans laquelle au moins une face de cette feuille est réticulée sur une épaisseur de 0,1 à 1 µm.

3. Feuille de polychlorure de vinyle réticulée selon les revendications 1 et 2, dans laquelle le degré de chloration n'est pas supérieur à 30%.

4. Feuille de polychlorure de vinyle réticulée selon l'une quelconque des revendications 1 à 3, dans laquelle le retrait thermique de cette feuille n'est pas supérieur à 0,5%.

5. Feuille de polychlorure de vinyle réticulée selon l'une quelconque des revendications 1 à 4, dans laquelle la face réticulée est partiellement fluorée.

6. Feuille de polychlorure de vinyle réticulée selon la revendication 5, dans laquelle le nombre d'atomes de fluor de la face réticulée n'est pas inférieur à 10% des atomes de carbone.

7. Procédé de fabrication d'une feuille de polychlorure de vinyle réticulée consistant à traiter thermiquement une feuille de polychlorure de vinyle telle que le retrait thermique de cette feuille n'est pas supérieur à 1% et à soumettre cette feuille à une décharge luminescente sous une pression de 1,3 Pa à 1,3 kPa en utilisant une fréquence de 1 à 1000 kilo-cycles.

8. Procédé selon la revendication 7, dans lequel cette décharge luminescente est effectuée dans un mélange gazeux comprenant l'oxyde de carbone et du tétrafluorure de carbone.

9. Poches à infusion et poches à sang fabriquées en utilisant des feuilles de polychlorure de vinyle selon l'une quelconque des exemples 1 à 6.

10. Utilisation de la poche à sang selon la revendication 9, dans laquelle au moins la face interne de la poche est traitée par du plasma froid et contient un agent anti-coagulant comprenant du citrate de sodium dans la poche.

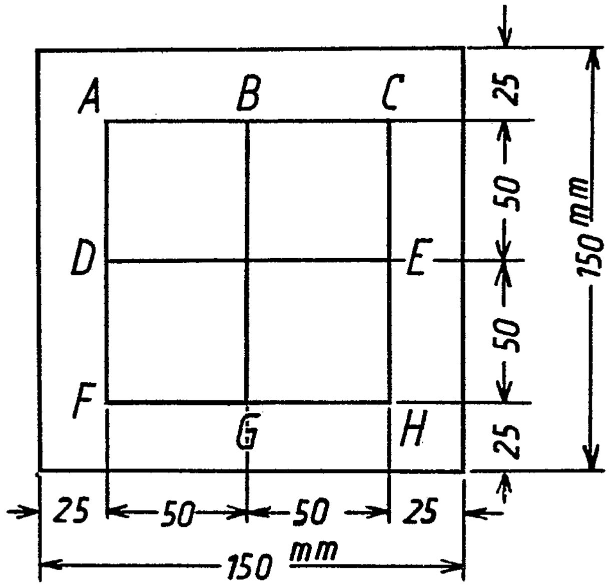


Fig.1

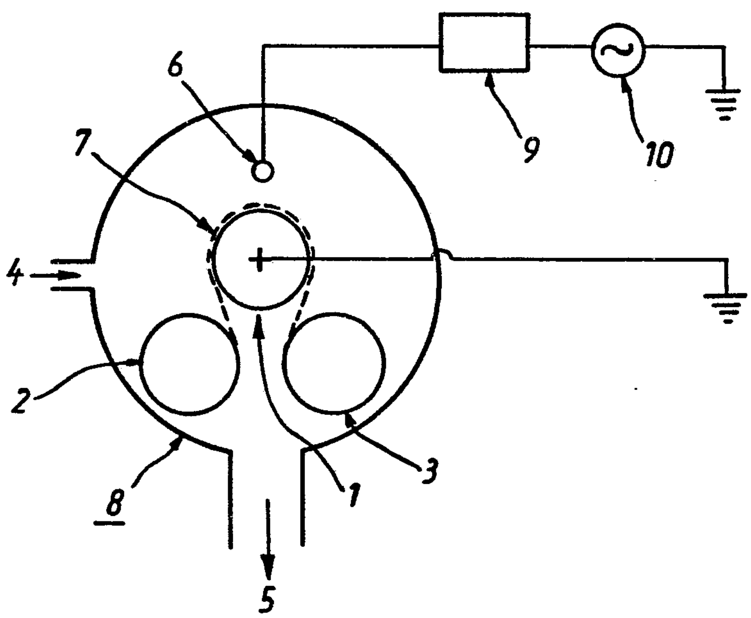


Fig.2

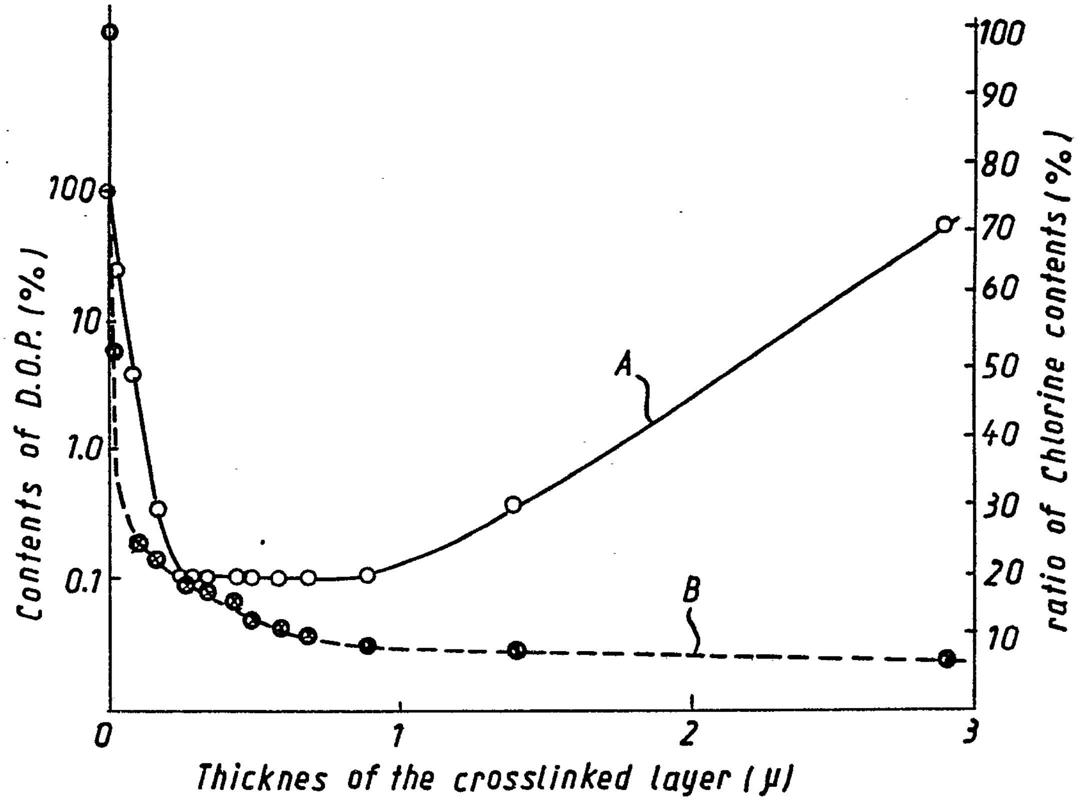

100
10
1.0
0.1
Contents of D.O.P. (%)
100
90
80
70
60
50
40
30
20
10
ratio of Chlorine contents (%)
A
B
0
1
2
3
Thicknes of the crosslinked layer (μ)


Fig. 3

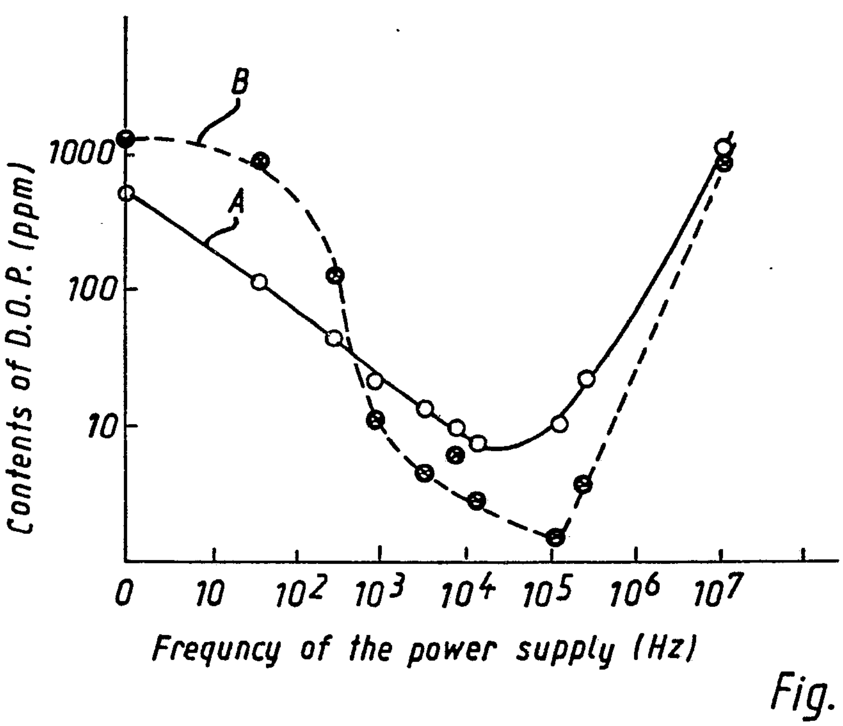

1000
100
10
Contents of D.O.P. (ppm)
B
A
0
10
$10^2$
$10^3$
$10^4$
$10^5$
$10^6$
$10^7$
Frequncy of the power supply (Hz)


Fig. 4